# EUROPEAN PATENT APPLICATION

(11) **EP 2 028 492 A1**
(43) Date of publication of application: **25.02.2009**
(21) Application number: 07744982.5
(22) Date of filing: 05.06.2007
(51) Int. Cl.: G01N 33/574

(54) **TUMOR MARKER AND METHOD FOR DETERMINATION OF THE OCCURRENCE OF CANCEROUS DISEASE**

(30) Priority: 05.06.2006 JP 2006156248
(71) Applicant: Shimadzu Corporation, Kyoto-shi Kyoto 604-8511 (JP); Osaka University, Suita-shi Osaka 565-0871 (JP)
(72) Inventor: WATANABE, Makoto, Kyoto-shi Kyoto 604-8511 (JP); NISHIMURA, Osamu, Kyoto-shi Kyoto 604-8511 (JP); MATSUBARA, Toshiya, Kyoto-shi Kyoto 604-8511 (JP); TAKEMASA, Ichiro, Suita-shi Osaka 565-0871 (JP); MONDEN, Morito, Suita-shi Osaka 565-0871 (JP); NAGAI, Katsuya, Osaka 565-0871 (JP); MATSUURA, Nariaki, Suita-shi Osaka 565-0871 (JP)
(74) Representative: Wilhelms · Kilian & Partner Patentanwälte
(86) International application number: PCT/JP2007/061690
(87) International publication number: WO 2007/142347

(57) **Abstract**

The present invention provides a tumor marker and a method capable of identifying the morbidity of colon cancer. A tumor marker including a protein identified from a colon cancer tissue. A method for identify the morbidity of colon cancer using the tumor marker. The method includes: measuring the level of the protein in a sample derived from a person of interest who should be examined to identify the morbidity of colon cancer; and comparing the measured level to the normal level of the protein, wherein a higher or lower measured level than the normal level is used as one indicator indicating that there is a high possibility that the person of interest has colon cancer.

## Description

### TECHNICAL FIELD

The present invention relates to a technique for clinical diagnosis, examination, and follow-up and classification. More specifically, the present invention relates to a tumor marker and a method to identify the morbidity of a cancer disease. Particularly, the present invention relates to a tumor marker for colon cancer and a method to identify the morbidity of colon cancer.

### BACKGROUND ART

As one method for diagnosis, examination, and follow-up of colon cancer, a blood test is performed. In a blood test, the level of some kind of protein (tumor marker) in the blood of a patient is measured to estimate the presence of advanced cancer. Tumor markers for colon cancer are disclosed in, for example, Anticancer Research, 2004, 24(4), 2519-2530 (Non-Patent Document 1) or the like. Currently, carcinoembryonic antigen (CEA) and CA19-9 can be mentioned as typical tumor markers. In a case where metastasis to the liver or the like occurs, the level of such a tumor marker is significantly increased. Further, US 2006/0019256 (COMPOSITIONS AND METHODS FOR TREATING AND DIAGNOSING CANCER) (Patent Document 1) discloses HLA-C, MBC2, RAB22A, MGC15429, and MHC class I antigen as cancer-associated proteins found as a result of the analysis of lung cancer-derived cultured cells (xenograft tumors). Further, US 2003/0211498 (TUMOR MARKERS IN OVARIAN CANCER) (Patent Document 2) discloses Tumor rejection antigen-1 as a uterus cancer marker gene.

Non-Patent Document 1: "Anticancer Research", 2004, vol. 24, No. 4, pp. 2519-2530
Patent Document 1: US2006/0019256
Patent Document 2: US2003/0211498

### DISCLOSURE OF THE INVENTION

### Object of the Invention

As described above, tumor markers such as carcinoembryonic antigen (CEA) and CA19-9 are used in a blood test for diagnosis, examination, and follow-up of colon cancer. However, the level of such a tumor marker is often normal in the stage of early cancer, and it is not unusual that the level of such a tumor marker is within a normal range even in the stage of advanced cancer. From such a viewpoint, there is a demand for finding a novel tumor marker highly specific to an affected part and suitable for early diagnosis.

It is to be noted that Patent Document 1 does not demonstrate that the disclosed cancer-associated proteins are associated with colon cancer, and Patent Document 2 does not demonstrate that the disclosed marker gene for uterus cancer is associated with colon cancer.

It is an object of the present invention to provide a tumor marker more highly specific to colon cancer. It is also an object of the present invention to provide a method capable of identifying the morbidity of colon cancer.

### Summary of the Invention

The present inventors have identified proteins showing a certain amount or more of difference in their abundance between tumor and normal tissue samples derived from the large-intestinal mucosal epithelia of colon cancer patients using the NBS method as a proteome analysis method. The NBS method is an excellent method for relative quantitation of protein/peptide using 2-nitrobenzenesulfenyl chloride. A proteome analysis method using the NBS method is fundamentally different from the most versatile proteome analysis method using two-dimensional electrophoresis in separation system and detection principles. Therefore, it is expected that it is possible to find proteins as candidates for markers different from a group of proteins previously reported as candidates for kidney cancer markers.

The present invention includes the following aspects.

The following (1) and (2) relate to a tumor marker for colon cancer.

The following (1) relates to a tumor marker for colon cancer including a protein expressed at a higher-than-normal level in cancer patients.
(1)
   A tumor marker for colon cancer including at least one protein selected from the group consisting of:
   6-phosphogluconolactonase; Alpha1 acid glyco protein; Alpha-actinin 1; Apurinic endonuclease; Calumenin; Chaperonin; Clathrin heavy chain 1; Clathrin light polypeptide A; c-myc binding protein; Complement factor H; Cysteine rich intestinal protein 1; F-box protein 40; Fibrinogen gamma; Fk506 Binding Protein Pkbp Mutant R42kH87V COMPLEX WITH Immunosuppressant Fk506; Guanine nucleotide binding protein (G protein), beta polypeptide 2-like 1; Heat shock 70kD protein 9B; Heparan sulfate proteoglycan 2; HLA-C; hypothetical protein FLJ38663; L-plastin polypeptide; MBC2; Migration inhibitory factor-related protein 14 variant E; Mitogen inducible gene; Proteasome subunit p58; RAB18, member RAS oncogene family; RAB22A; Radixin; RAN, member RAS oncogene family; Rhodanese;thiosulfate sulfurtransferase; Ribosomal protein L13; Ribosomal protein L27a; Ribosomal protein L4; Ribosomal protein S18; Ribosomal protein S29; Ribosome binding protein 1; S adenosylhomocysteine hydrolase; Solute carrier family 25 (mitochondrial carrier; adenine nucleotide translocator), member 5; Solute carrier family 3(activator of dibasic and neutral amino acid transport), member 2; Splicing factor 3B, subunit 3; Splicing factor, arginine/serine-rich 3 (SRp20); U5 snRNP-specific protein, 116 kD; Ubiquitin isopeptidase T; Vitronectin; XTP3 transactivatied protein A; Galectin 1; Reticulocalbin 1; Vimentin; ESP-2 (zyxin);
   Protein tyrosine phosphatase receptor type C; Protein tyrosine phosphatase, receptor type, alpha; orosomucoid 2; Tumor rejection antigen 1; glycyl-tRNA synthetase; TLS protein; Ribonuclease RNase A family 3; and heterogeneous nuclear ribonucleoprotein H2.

   The following (2) relates to a tumor marker for colon cancer including a protein expressed at a lower-than-normal level in colon cancer patients.
(2)
   A tumor marker for colon cancer including a protein selected from the group consisting of:
   ADP-ribosylation factor-like 10C; aldehyde dehydrogenase2; Alpha-actinin 4; Annexin A2 isoform 2; ATP synthase, H+ transporting, mitochondrial F0 complex, subunit d isoform a; ATP-binding cassette transporter family A member 12; Calnexin; Carbonic Anhydrase Form B; Carbonyl reductase 1; Cathepsin S; cysteine rich protein 1; Dynein light chain 1; Endoplasmic-reticulum-lumenal protein 28; Enoyl coenzyme hydrase,shart chain; Eukaryotic translation elongation factor 2; Filamin B; gelsolin isoform a; Clucosamine-fructose-6-phasphate aminotransferase; GTP-binding protein Rab3B; Haptoglobin; Heterogeneous nuclear ribonucleoprotein A2; Hydroxymethylglutaryl-CoA synthase, mitochondrial; Isocitrate dehydrogenase 1; Lymphocyte cytosolic protein 1; Major vault protein; MGC15429 protein; MHC class I antigen; Myosin, heavy polypeptide 14; Myozenin 3; NADH Ubiquinone oxidoreductase subunit B13; Normal mucosa of esophagus specific 1; Olfactomedin 4; phosphoenolpyruvate calboxykinase 2; Phosphoglycerate mutase 1; Proline arginine-rich end leucine-rich repeat protein precursor; Protein kinase C and casein kinase substrate in neurons 2; Protein P97; Pyridoxine 5'-phosphate oxidase; Raf kinase inhibitor protein; Ras associated protein Rab5B; Retinoblastoma binding protein 4; succinate dehydrogenase complex,subunit A,flavoprotein; Thioredoxin domain containing 5; TNRC15 protein;
   Collagen, type XIV, alpha 1, and Desmoglein 2.

   The following (3) to (6) relate to a method for identifying the morbidity of colon cancer. In the present invention, the term "morbidity" widely refers to a state of having a disease, and the phrase "identifying the morbidity of colon cancer" includes performing detection, diagnosis, monitoring, staging, and prognostic evaluation of colon cancer.
   The following (3) and (4) relate to a method for identifying the morbidity of colon cancer by using the tumor marker for colon cancer according to the above (1).
(3)
   A method for identifying the morbidity of colon cancer by using, as a tumor marker for colon cancer, at least one protein selected from the group consisting of:
   6-phosphogluconolactonase; Alpha1 acid glyco protein; Alpha-actinin 1; Apurinic endonuclease; Calumenin; Chaperonin; Clathrin heavy chain 1; Clathrin light polypeptide A; c-myc binding protein; Complement factor H; Cysteine rich intestinal protein 1; F-box protein 40; Fibrinogen gamma; Fk506 Binding Protein Fkbp Mutant R42kH87V COMPLEX WITH Immunosuppressant Fk506; Guanine nucleotide binding protein (G protein), beta polypeptide 2-like 1; Heat shock 70kD protein 9B; Heparan sulfate proteoglycan 2; HLA-C; hypothetical protein FLJ38663; L-plastin polypeptide; MBC2; Migration inhibitory factor-related protein 14 variant E; Mitogen inducible gene; Proteasome subunit p58; RAB18, member RAS oncogene family; RAB22A; Radixin; RAN, member RAS oncogene family; Rhodanese;thiosulfate sulfurtransferase; Ribosomal protein L13; Ribosomal protein L27a; Ribosomal protein L4; Ribosomal protein S18; Ribosomal protein S29; Ribosome binding protein 1; S adenosylhomocysteine hydrolase; Solute carrier family 25 (mitochondrial carrier; adenine nucleotide translocator), member 5; Solute carrier family 3(activator of dibasic and neutral amino acid transport), member 2; Splicing factor 3B, subunit 3; Splicing factor, arginine/serine-rich 3 (SRp20); U5 snRNP-specific protein, 116 kD; Ubiquitin isopeptidase T; Vitronectin; XTP3 transactivatied protein A; Galectin 1; Reticulocalbin 1; Vimentin; ESP-2 (zyxin);
   Protein tyrosine phosphatase receptor type C; Protein tyrosine phosphatase, receptor type, alpha; orosomucoid 2; Tumor rejection antigen 1; glycyl-tRNA synthetase; TLS protein; Ribonuclease RNase A family 3; and heterogeneous nuclear ribonucleoprotein H2.
(4)
   The method for identifying the morbidity of colon cancer according to the above (5), including:
   measuring the level of the protein in a sample derived from a person of interest who should be examined to identify the morbidity of colon cancer; and
   comparing the measured level to the normal level of the protein,
   wherein a higher measured level than the normal level is used as one indicator indicating that there is a high possibility that the person of interest has colon cancer.

   The normal level of the protein is not particularly limited as long as it is the level of the protein in a non-cancerous sample served as a control for a cancerous sample, and examples thereof include the level of the protein in a normal sample derived from a healthy person and the level of the protein in a non-cancerous normal sample derived from a patient having a cancer disease.
   The method for identifying the morbidity of colon cancer according to the above (4), wherein the sample is blood serum or urine, and wherein the level of the protein is measured by an examination based on biospecific affinity.
   The following (5) and (6) relate to a method for identifying the morbidity of colon cancer by using the tumor marker for colon cancer according to the above (2).
(5)
   A method for identifying the morbidity of colon cancer by using, as a tumor marker for colon cancer, a protein selected from the group consisting of ADP-ribosylation factor-like 10C; aldehyde dehydrogenase2; Alpha-actinin 4; Annexin A2 isoform 2; ATP synthase, H+ transporting, mitochondrial F0 complex, subunit d isoform a; ATP-binding cassette transporter family A member 12; Calnexin; Carbonic Anhydrase Form B; Carbonyl reductase 1; Cathepsin S; cysteine rich protein 1; Dynein light chain 1; Endoplasmic-reticulum-lumenal protein 28; Enoyl coenzyme hydrase, short chain1; Eukaryotic translation elongation factor 2; Filamin B; gelsolin isoform a; Glucosamine-fructose-6-phosphate aminotransferase; GTP-binding protein Rab3B; Haptoglobin; Heterogeneous nuclear ribonucleoprotein A2; Hydroxymethylglutaryl-CoA synthase, mitochondrial; Isocitrate dehydrogenase 1; Lymphocyte cytosolic protein 1; Major vault protein; MGC15429 protein; MHC class I antigen; Myosin, heavy polypeptide 14; Myozenin 3; NADH Ubiquinone oxidoreductase subunit B13; Normal mucosa of esophagus specific 1; Olfactomedin 4; phosphoenolpyruvate calboxykinase 2; Phosphoglycerate mutase 1; Proline arginine-rich end leucine-rich repeat protein precursor; Protein kinase C and casein kinase substrate in neurons 2; Protein P97; Pyridoxine 5'-phosphate oxidase; Raf kinase inhibitor protein; Ras associated protein Rab5B; Retinoblastoma binding protein 4; succinate dehydrogenase complex, subunit A, flavopratein; Thioredoxin domain containing 5; TNRC15 protein;
   Collagen, type XIV, alpha 1, and Desmoglein 2.
(6)
   The method for identifying the morbidity of colon cancer according to the above (6), including:
   measuring the level of the protein in a sample derived from a person of interest who should be examined to identify the morbidity of colon cancer; and
   comparing the measured level to the normal level of the protein,
   wherein a lower measured level than the normal level is used as one indicator indicating that there is a high possibility that the person of interest has colon cancer.

   The normal level of the protein is not particularly limited as long as it is the level of the protein in a non-cancerous sample served as a control for a cancerous sample, and examples thereof include the level of the protein in a normal sample derived from a healthy person and the level of the protein in a non-cancerous normal sample derived from a patient having a cancer disease.
   The method for identifying the morbidity of colon cancer according to the above (6), wherein the sample is blood serum or urine, and wherein the level of the protein is measured by an examination based on biospecific affinity.
   The present invention is also directed to a drug composition for treatment of colon cancer described below in (7) and (8). The treatment of colon cancer includes killing colon cancer cells and suppressing the growth of colon cancer cells.
(7)
   A drug composition to be supplied to colon cancer cells to induce killing of colon cancer cells and/or reaction promoting suppression of the growth of colon cancer cells, said drug composition including at least one antibody to be immunospecifically bound to the tumor marker according to the above (1).
(8)
   A drug composition to be supplied to colon cancer cells in an immunostimulating amount to promote immune response, said drug composition including the tumor marker according to the above (1).
   The drug composition according to the above (7) or (8) can be regarded as a latent therapeutic drug for treatment of colon cancer or tdrug composition according to the above (7) or (8) can be used as a therapeutic drug for treatment of colon cancer.
   According to the present invention, it is possible to provide a tumor marker more highly specific to colon cancer and a method capable of identifying the morbidity of colon cancer.

### BRIEF DESCRIPTION OF THE DRAWINGS

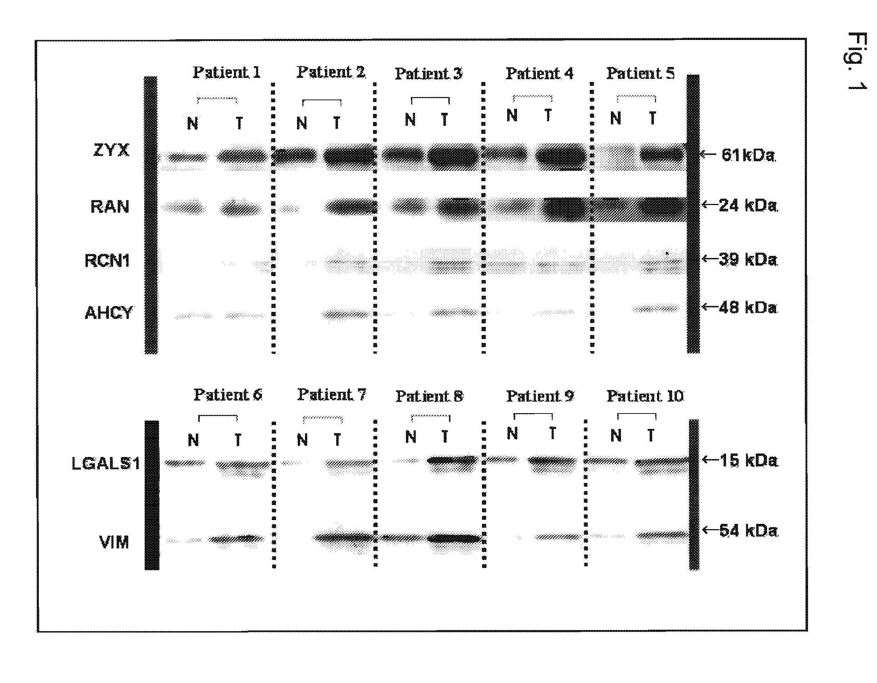

Fig. 1 shows the result of western blotting analysis of 6 proteins according to the present invention showing higher expression in colon cancer tissues, which was performed using clinical samples (cancerous and non-cancerous part of the large-intestinal mucosal epithelial tissues of colon cancer patients).

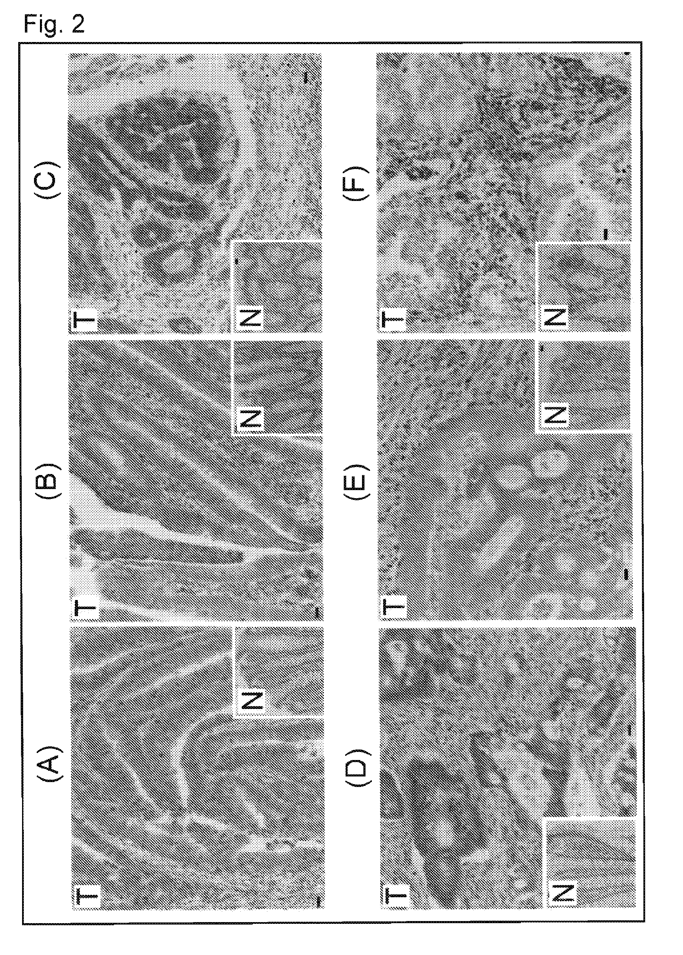

Fig. 2 shows the result of validation analysis of 6 proteins according to the present invention showing higher expression in colon cancer tissues, which was performed by immunohistochemical staining of clinical samples (cancerous and non-cancerous part of the large-intestinal mucosal epithelial tissues of colon cancer patients).

### MODES FOR CARRYING OUT THE INVENTION

### <Tumor Marker>

The present invention provides a tumor marker for colon cancer.

Proteins provided as tumor markers in the present invention were extracted from cancerous and non-cancerous part of the large-intestinal mucosal epithelial tissues sampled from colon cancer patients, and identified by a proteome analysis technique including isotopic labeling method (NBS method) using 2-nitrobenzenesulfenyl chloride (NBSCI) and HPLC separation. The NBS method is a method capable of determining a relative difference in protein contest between two protein samples in different states, performed by: one of the two protein samples in different states is modified with a heavy reagent (2-nitro[¹³C₆]benzenesulfenyl chloride) and the other is modified with a light reagent (2-nitro[¹²C₆]benzenesulfenyl chloride), and then the thus obtained NBS-modified protein samples are mixed together and subjected to appropriate treatment, such as tryptic digestion, selected by those skilled in the art to quantify a difference in peptide content by a mass spectrometer. The NBS method is described in Rapid Commun. Mass Spectrom., 2003, 17, 1642-1650 and WO 2004/002950.

Two-dimensional electrophoresis conventionally widely used has difficulty in separating and detecting proteins having a basic isoelectric point and proteins having a large molecular weight of about 100 KDa. However, the NF3S method used in the present invention can be carried out without constraints on molecular weight and isoelectric point because targets to be directly analyzed are peptide fragments obtained by tryptic digestion of a protein contained in tissues and separation is carried out by HPLC. For this reason, there is a possibility that findings different from those previously reported can be obtained.

These proteins may be isolated and purified by any protein purification techniques selected by those skilled in the art. Examples of such techniques include chromatography (e.g., ion-exchange chromatography, affinity chromatography, or size exclusion column chromatography), centrifugal separation, difference in solubility, and electrophoresis.

Among the proteins provided as tumor markers provided in the present invention, the present invention provides the following proteins are expressed at a higher-than-normal level in cancer patients:
6-phosphogluconolactonase; Alpha1 acid glyco protein; Alpha-actinin 1; Apurinic endonuclease; Calumenin; Chaperonin; Clathrin heavy chain 1; Clathrin light polypeptide A; c-myc binding protein; Complement factor H; Cysteine rich intestinal protein 1; F-box protein 40; Fibrinogen gamma; Fk506 Binding Protein Fkbp Mutant R42kH87V COMPLEX WITH Immunosuppressant Fk506; Guanine nucleotide binding protein (G protein), beta polypeptide 2-like 1; Heat shock 70kD protein 9B; Heparan sulfate proteoglycan 2; HLA-C; hypothetical protein FLJ38663; L-plastin polypeptide; MBC2; Migration inhibitory factor-related protein 14 variant E; Mitogen inducible gene; Proteasome subunit p58; RAB318, member RAS oncogene family; RAB22A; Radixin; RAN, member RAS oncogene family; Rhodanese;thiosulfate sulfurtransferase; Ribosomal protein L13; Ribosomal protein L27a; Ribosomal protein L4; Ribosomal protein S18; Ribosomal protein S29; Ribosome binding protein 1; S adenosylhomocysteine hydrolase; Solute carrier family 25 (mitochondrial carrier; adenine nucleotide translocator), member 5; Solute carrier family 3(activator of dibasic and neutral amino acid transport), member 2; Splicing factor 3B, subunit 3; Splicing factor, arginine/serine-rich 3 (SRp20); U5 snRNP-specific protein, 116 kD; Ubiquitin isopeptidase T; Vitronectin; XTP3 transactivatied protein A; Galectin 1; Reticulocalbin 1; Vimentin; ESP-2 (zyxin);

Protein tyrosine phosphatase receptor type C; Protein tyrosine phosphatase, receptor type, alpha; orosomucoid 2; Tumor rejection antigen 1; glycyl-tRNA synthetase; TLS protein; Ribonuclease RNase A family 3; and heterogeneous nuclear ribonucleoprotein H2.

The above-mentioned proteins tend to be expressed at an about 50% or more higher, preferably 100% or more higher level in cancerous part than in non-cancerous part in, for example, about 50% or more of the colon cancer patients.

Further, among the above-mentioned proteins, Protein tyrosine phosphatase receptor type C; Protein tyrosine phosphatase, receptor type, alpha; orosomucoid 2; Tumor rejection antigen 1; glycyl-tRNA synthetase; TLS protein; Ribonuclease RNase A family 3; and heterogeneous nuclear ribonucleoprotein H2 are specifically expressed in cancerous part (or the levels of the following proteins in non-cancerous part are below their detection limit):

Among the proteins provided as tumor markers provided in the present invention, the present invention provides the following proteins are expressed at a lower-than-normal level in cancer patients:

ADP-ribosylation factor-like 10C; aldehyde dehydrogenase2; Alpha-actinin 4; Annexin A2 isoform 2; ATP synthase, H+ transporting, mitochondrial F0 complex, subunit d isoform a; ATP-binding cassette transporter family A member 12; Calnexin; Carbonic Anhydrase Form B; Carbonyl reductase 1; Cathepsin S; cysteine rich protein 1; Dynein light chain 1; Endoplasmic-reticulum-lumenal protein 28; Enoyl coenzyme hydrase,short chain; Eukaryotic translation elongation factor 2; Filamin B; gelsolin isoform a; Glucosamine-fructose-6-phosphate aminotransferase; GTP-binding protein Rab3B; Haptoglobin; Heterogeneous nuclear ribonucleoprotein A2; Hydroxymethylglutaryl-CoA synthase, mitochondrial; Isocitrate dehydrogenase 1; Lymphocyte cytosolic protein 1; Major vault protein; MHC15429 protein; MHC class I antigen; Myosin, heavy polypeptide 14; Myozenin 3; NADH Ubiquinone oxidoreductase subunit B13; Normal mucosa of esophagus specific 1; Olfactomedin 4; phosphoenolpyruvate calboxykinase 2; Phosphoglycerate mutase 1; Proline arginine-rich end leucine-rich repeat protein precursor; Protein kinase C and casein kinase substrate in neurons 2; Protein P97; Pyridoxine 5'-phosphate oxidase; Raf kinase inhibitor protein; Ras associated protein Rab5B; Retinoblastoma binding protein 4; succinate dehydrogenase complex,subunit A,flavoprotein; Thioredoxin domain containing 5; TNRC15 protein;
Collagen, type XIV, alpha 1, and Desmoglein 2.

The above-mentioned proteins tend to be expressed at an about 50% or more higher, preferably 100% or more higher level in non-cancerous part than in cancerous part in, for example, about 50% or more of the colon cancer patients.

Further, among the above-mentioned proteins, Collagen, type XIV, alpha 1, and Desmoglein 2 are specifically expressed in non-cancerous part (or the levels of these proteins in cancerous part are below their detection limit).

### <Method for Identifying Morbidity of Colon Cancer>

The present invention also provides a method for identifying the morbidity of colon cancer by using at least one protein selected from the above-mentioned group of proteins as a tumor marker.

According to the method of the present invention, a sample derived from a person of interest who should be examined to identify the morbidity of colon cancer is provided, and the level of at least one protein selected from the above-mentioned group of proteins in the sample is measured. The measured level is compared to a normal level. The normal level is the level of at least one protein selected from the above-mentioned group of proteins in a sample served as a control for a cancerous sample. Here, the sample served as a control for a cancerous sample is not particularly limited as long as it is a non-cancerous sample, and examples of such a non-cancerous sample include a sample derived from a healthy person and a normal sample derived from a patient having a cancer disease.

In the present invention, the sample derived from a person of interest who should be examined to identify the morbidity of colon cancer is not particularly limited, and examples thereof include cells or tissues, body fluids, and tissue extracts. The cells or tissues also include tissue biopsy materials, autopsy materials, and tissue slices and tissue extracts thereof. Particularly, as the samples, cells, tissues, and tissue extracts of large intestine-derived samples may be mentioned. Examples of the large intestine-derived samples include mucous epithelium, lamina propria mucosae, muscularis mucosae, submucosa, muscularis propria, and serous membrane. Examples of the body fluids include blood, urine, and body secretion. The term "blood" includes whole blood, blood plasma, and blood serum. The tissue extract refers to one obtained by homogenizing or solubilizing a tissue by a method well known to those skilled in the art. Among these samples exemplified above, blood serum and/or urine are/is preferred.

The measured level of the tumor marker in a sample containing cells or tissues, body fluids, and/or a tissue extract of a person of interest is preferably compared to that in a non-cancerous sample containing the same kind(s) of cells or tissues, body fluids, and/or a tissue extract.

In identifying the morbidity of colon cancer, the tumor marker is used for the following purposes. The tumor marker in a tissue is used as, for example, a target in cancer diagnosis or prognosis using tissue slices (which is carried out by, for example, immunohistochemical staining or mass imaging) or in PET diagnosis (which is carried out by, for example, labeling the tumor marker with a radioactive probe) or a drug target in medical treatment (see <Drug Composition> described later). On the other hand, the tumor marker in blood is used as, for example, a target to be quantitatively measured in cancer diagnosis or prognosis.

In a case where the protein group is a group consisting of:
6-phosphogluconolactonase; Alpha1 acid glyco protein; Alpha-actinin 1; Apurinic endonuclease; Calumenin; Chaperonin; Clathrin heavy chain 1; Clathrin light polypeptide A; c-myc binding protein; Complement factor H; Cysteine rich intestinal protein 1.; F-box protein 40; Fibrinogen gamma; Fk506 Binding Protein Fkbp Mutant R42kH87V COMPLEX WITH Immunosuppressant Fk506; Guanine nucleotide binding protein (G protein), beta polypeptide 2-like 1; Heat shock 70kD protein 9B; Heparan sulfate proteoglycan 2; HLA-C; hypothetical protein FLJ38663; L-plastin polypeptide; MBC2; Migration inhibitory factor-related protein 14 variant E; Mitogen inducible gene; Proteasome subunit p58; RAB18, member RAS oncogene family; RAB22A; Radixin; RAN, member RAS oncogene family; Rhodanese;thiosulfate sulfurtransferase; Ribosomal protein L13; Ribosomal protein L27a; Ribosomal protein L4; Ribosomal protein S18; Ribosomal protein S29; Ribosome binding protein 1; S adenosylhomocysteine hydrolase; Solute carrier family 25 (mitochondrial carrier; adenine nucleotide translocator), member 5; Solute carrier family 3(activator of dibasic and neutral amino acid transport), member 2; Splicing factor 3B, subunit 3; Splicing factor, arginine/serine-rich 3 (SRp20); U5 snRNP-specific, protein, 116 kD; Ubiquitin isopeptidase T; Vitronectin; XTP3 transactivatied protein A; Galectin 1; Reticulocalbin 1; Vimentin; ESP-2 (zyxin);
Protein tyrosine phosphatase receptor type C; Protein tyrosine phosphatase, receptor type, alpha; orosomucoid 2; Tumor rejection antigen 1; glycyl-tRNA synthetase; TLS protein; Ribonuclease RNase A family 3; and heterogeneous nuclear ribonucleoprotein H2,
a higher measured level than the normal level of the protein may be used as one indicator indicating that there is a high possibility that a person of interest has colon cancer. An increase degree in the measured level may tentatively be such that the measured level is about 50% or more higher, preferably about 100% or more higher than the normal level.

Among these proteins, Protein tyrosine phosphatase receptor type C; Protein tyrosine phosphatase, receptor type, alpha; orosomucoid 2; Tumor rejection antigen 1; glycyl-tRNA synthetase; TLS protein; Ribonuclease RNase A family 3; and heterogeneous nuclear ribonucleoprotein H2 are proteins specifically expressed in colon cancer tissues. Therefore, the presence of such a protein may be used as one indicator indicating that there is a high possibility that a person of interest has colon cancer.

In a case where the protein group is a group consisting of:
ADP-ribosylation factor-like 10C; aldehyde dehydrogenase2; Alpha-actinin 4; Annexin A2 isoform 2; ATP synthase, H+ transporting, mitochondrial F0 complex, subunit d isoform a; ATP-binding cassette transporter family A member 12; Calnexin; Carbonic Anhydrase Form B; Carbonyl reductase 1; Cathepsin S; cysteine rich protein 1; Dynein light chain 1; Endoplasmic-reticulum-lumenal protein 28; Enoyl coenzyme hydras,short chain1; Eukaryotic translation elongation factor 2; Filamin B; gelsolin isoform a; Glucosamine-fructose-6-phosphate aminotransferase; GTP-binding protein Rab3B; Haptoglobin; Heterogeneous nuclear ribonucleoprotein A2; Hydroxymethylglutaryl-CoA synthase, mitochondrial; Isocitrate dehydrogenase 1; Lymphocyte cytosolic protein 1; Major vault protein; MGC15429 protein; MHC class I antigen; Myosin, heavy polypeptide 14; Myozenin 3; NADH Ubiquinone oxidoreductase subunit B13; Normal mucosa of esophagus specific 1; Olfactomedin 4; phosphoenolpyruvate calboxykinase 2; Phosphoglycerate mutase 1; Proline arginine-rich end leucine-rich repeat protein precursor; Protein kinase C and casein kinase substrate in neurons 2; Protein P97; Pyridoxine 5'-phosphate oxidase; Raf kinase inhibitor protein; Ras associated protein Rab5B; Retinoblastoma binding protein 4; succinate dehydrogenase complex,subunit A,flavoprotein; Thioredoxin domain containing 5; TNRC15 protein;
Collagen, type XIV, alpha 1, and Desmoglein 2,
a lower measured level than the normal level of the protein ,may be used as one indicator indicating that there is a high possibility that a person of interest has colon cancer. A decrease degree in the measured level may tentatively be such that the normal level is about 50% or more higher, preferably about 100% or more higher than the measured level.

Among these proteins, Collages, type XIV, alpha 1, and Desmoglein 2 are proteins specifically expressed in normal tissues (non-cancerous tissues). Therefore, the absence of such a protein may be used as one indicator indicating that there is a high possibility that a person of interest has colon cancer.

The level of the protein is preferably measured by an examination based on biospecific affinity. Such an examination based on biospecific affinity is well known to those skilled in the art, and is not particularly limited. However, an immunoassay is preferably used. Specific examples of an immunoassay include competitive and noncompetitive assay systems such as western blotting, radioimmunoassay, ELISA, sandwich immunoassay, immunoprecipitation, precipitation reaction, gel diffusion precipitin reaction, immunodiffusion, aggregation measurement, complement binding assay, immunoradiometric assay, fluorescence immunoassay, and protein A immunoassay. Such an immunoassay is carried out to detect the presence of an antibody bound to a tumor marker in the sample of a person of interest. More specifically, such an immunoassay is carried out by bringing the sample into contact with an antibody in an assay medium under conditions where the tumor marker protein and its antibody can form an immune complex. A more specific immunoassay protocol may be easily selected by those skilled in the art.

As described above, the level of the protein is preferably measured by an examination based on biospecific affinity, but may be measured by another protein quantitation method. For example, the NBS method described above is an excellent quantitation method. In this case, the level of the protein can be measured by determining a difference in the abundance of the protein between the sample of a person of interest and an appropriate control sample such as a sample containing a known level of the protein or a normal sample.

According to the method of the present invention, the tumor marker according to the present invention may be measured alone or in combination with any other tumor marker. Therefore, the method according to the present invention may include measuring the level of another tumor marker in addition to measuring the level of the tumor marker according to the present invention.

The tumor marker according to the present invention may be used for detection, diagnosis, monitoring, staging, and prognostic evaluation of colon cancer, and is preferably used to examine tumor kinetics. For example, in the case of treatment of a tumor by chemical therapy or radiation therapy, the tumor marker may be used to determine how much effect the therapy has had. Further, in the case that the excisional surgery is operated for a tumor showing a high tumor marker level, the tumor marker may be used for postoperative follow-up.

### <Drug Composition>

The present invention is also directed to a drug composition for treatment of colon cancer using the tumor marker according to the present invention including a protein expressed at a higher-than-normal level in colon cancer patients.

One embodiment of the present invention provides a drug composition to be supplied to cancer cells to induce killing of cancer cells and/or reaction promoting suppression of the growth of cancer cells, the drug composition including at least one antibody to be immunospecifically bound to the tumor marker according to the present invention. The term "antibody" includes polyclonal antibodies, monoclonal antibodies, and antibodies prepared by molecular biologic techniques. Here, the antibody widely refers to a material which has immunospecifical bounding ability. For example, antibody fragments and antibody fusion proteins may also be used. In each case, such an antibody is prepared by a method well known to those skilled in the art.

Another embodiment of the present invention provides a drug composition to be supplied to cancer cells in an immunostimulating amount to promote immune response, the drug composition including the tumor marker according to the present invention. Here, the immunostimulating amount refers to the amount of an antigen capable of inducing desired immune response for treatment of cancer, and is determined by a method well known to those skilled in the art. By using such a drug composition, it is possible to carry out treatment of cancer, known as so-called cancer vaccine therapy, by a method well known to those skilled in the art.

The above drug composition according to the present invention includes, as an active ingredient, the above-described antibody or tumor marker, but may further include a pharmaceutically acceptable diluent, carrier, excipient, or the like. The drug composition according to the present invention can be regarded as a latent therapeutic drug for use in treatment of colon cancer or can be used as a therapeutic drug for use in treatment of colon cancer.

### EXAMPLE

Hereinbelow, the present invention will be concretely described with reference to the following examples, but is not limited to these examples.

### <Example 1: Protein Expression Analysis by NBS Method>

Normal tissues (i.e., non-cancerous part of large-intestinal mucosal epithelial tissues) and cancerous tissues (i.e., cancerous part of large-intestinal mucosal epithelial tissues) were sampled from colon cancer patients. Each of the tissues was homogenized in a solubilizing buffer A (50 mM Tris-HCl (pH 8.0), 100 mM NaCl, 10 mM EDTA, protease inhibitor (aprotinin, PMSF, Leupeptin) solution), and was then ultracentrifuged at 100000 × G (4°C, 1 hour) to obtain a supernatant solution as a soluble fraction. Then, the precipitate obtained by centrifugation was again homogenized in a solubilizing buffer B (9M urea, 2 w/v% CHAPS, 10 mM EDTA, protease inhibitor (aprotinin, PMSF, Leupeptin) solution), and was then ultracentrifuged at 100000 × G (4°C, 1 hour) to obtain a supernatant solution as an insoluble fraction.

Extracted proteins contained in these soluble and insoluble fractions were treated with NBS reagents to carry out protein expression analysis. The treatment using NBS reagents was carried out according to a recommended protocol supplied with ¹³C NBS Stable Isotope Labeling Kit-N (manufactured by Shimadzu Corporation) to label the extracted proteins with stable isotopes. More specifically, proteins extracted from the normal tissue were labeled with ¹²CNBS (Light NBS), and proteins extracted from the cancerous tissue were labeled with ¹³CNBS (Heavy NBS). Then, the thus obtained labeled proteins were mixed together, and the protein mixture was subjected to desalination, reduction, alkylation, and tryptic digestion according to the recommended protocol supplied with the kit. The thus obtained labeled peptides were concentrated according to the protocol supplied with the kit, and then the concentrated labeled peptides were subjected to separation by µHPLC using a C18 column and applied to an MS plate. The thus prepared sample was analyzed using a mass spectrometer Axima-CFR plus (manufactured by Shimadzu Corporation) to carry out relative quantitative analysis of each protein.

Peptide fragments appearing as a pair of peaks were subjected to MS/MS analysis using a tandem mass spectrometer Axima-QIT (manufactured by Shimadzu Corporation) to carry out protein identification.

In this way, 24 clinical samples were finally analyzed. As a result, proteins whose expression level in cancerous tissues were increased or decreased in 50% or more of all the samples were identified. The increase or decrease degree is based on the following level. Namely, in the case of peptides whose abundance in cancerous part was higher than that in non-cancerous part, peptides whose abundance in cancerous part was 50% or more higher than that in non-cancerous part (i.e., peptides whose abundance in cancerous part was 150% or more of that in non-cancerous part) were selected. On the other hand, in the case of peptides whose abundance in cancerous part was lower than that in non-cancerous part, peptides whose abundance in non-cancerous part was 50% or more higher than that in cancerous part (i.e., peptides whose abundance in cancerous part was less than 66% of that in non-cancerous part) were selected.

As a result, proteins listed in the following Tables 1 to 8 were identified. In Tables 1 to 8, "mean", "SEM", and "SD" respectively represent a mean value, a standard error, and a standard deviation of relative ratios (%) measured in all the clinical samples (i.e., the expression level (ratio) of a protein in cancerous part when the expression level of the protein in non-cancerous part was regarded as 100 (%)).

Tables 1. to 3 show the list of identified proteins (Up-regulated proteins) whose expression level in cancerous part was higher than that in non-cancerous part. For example, 6-phosphogluconolactonase (averaged expression level: about 2.16 times that in non-cancerous part), whose expression level was increased in 16 samples out of the 24 samples, or the like were identified. Table 4 is the list of proteins specifically (All or None) detected in cancerous part. In Tables 1 to 4, "150% ≤" represents the number of samples in which the abundance of a protein in cancerous part was 150% or more of that in non-cancerous part.

**Table 1**

| **Colon cancer-related proteins (Up-regulated proteins)** | Gene Symbol | Gene Map | 150%≦ | Mean | SEM | SD |
|---|---|---|---|---|---|---|
| 6-phosphogluconolactonase | PGLS | 19p13.2 | 16 | 216.6 | 46.9 | 187.8 |
| Alpha1 acid glyco protein | ORM1 | 9q34.1-q34.3 | 17 | 295.1 | 38.6 | 159.1 |
| Alpha-actinin 1 | ACTN2 | 1q42-q43, | 15 | 226.05 | 10.8 | 74.6 |
| Aporinic endonuclease | APEX1 | 14q11.2-q12 | 16 | 218.1 | 27.9 | 111.6 |
| Calumenin | CALM | 7q32 | 16 | 306.6 | 53.8 | 215.3 |
| Chaperonin | HSPD1 | 2q33.1 | 17 | 239.7 | 18.1 | 74.7 |
| Clathrin heavy chain 1 | CLTC | 17q11-qter | 14 | 212.7 | 16.1 | 60.3 |
| Clathrin light polypeptide A | CLTA | 9p13 | 18 | 398.7 | 59.6 | 253 |
| c-myc binding protein | MYCBP | 1 p33-p32.2 | 20 | 235.7 | 18.2 | 81.5 |
| Complement factor H | CFH | 1 q32 | 14 | 203.3 | 11.8 | 43.4 |
| Cysteine rich intestinal protein 1 | CRIP1 | 7q11.23 | 13 | 211.5 | 21.3 | 76.8 |
| F-box protein 40 | FBXO40 | 3q13.33 | 18 | 257.1 | 30.3 | 121.2 |
| Fibrinogen gamma | FGG | 4q28 | 17 | 240.2 | 16.6 | 68.3 |
| Fk506 Binding Protein Fkbp Mutant R42kH87V COMPLEX WITH Immunosuppressant Fk506 | FKBP1A FKBPLA | 20p13 | 15 | 257 | 53.8 | 208.4 |
| Guanine nucleotide binding protein (G protein), beta polypeptide 2-like 1 | MYCL1 | 1p34.2 | 18 | 229.5 | 18.3 | 77.7 |
| Heat shock 70kD protein 9B | HSPA9B | 5q31.1 | 17 | 312.9 | 44 | 181.5 |
| Heparan sulfate proteoglycan 2 | SDC2 | 8q22-q23 | 19 | 255.3 | 25.5 | 111.4 |

**Table 2**

| **Colon cancer-related proteins (Up-regulated proteins)** | Gene Symbol | Gene Map | 150%≦ | Mean | SEM | SD |
|---|---|---|---|---|---|---|
| HLA-C | HLA-C | 6p21.3 | 17 | 300.8 | 37.4 | 154.2 |
| hypothetical protein FLJ38663 | None | 12q24.31 | 14 | 202.7 | 15.8 | 58 |
| L-plastin polypeptide | LCP1 | 13q14.3 | 16 | 232.9 | 24.9 | 99.5 |
| MBC2 | - | 12q13.2 | 14 | 202 | 15.2 | 56.9 |
| Migration inhibitory factor-related protein variant E | S100A9 | 1q12-q22 | 19 | 335.9 | 73.7 | 321.3 |
| Mitogen inducible gene | PLEKHC1 | 14q22.1 | 12 | 193.1 | 11.6 | 40.3 |
| Proteasome subunit p58 | PSMD3 | 17q21.2 | 13 | 255.5 | 31.8 | 114.6 |
| RAB18, member RAS oncogene family | RAS18 | 10p12.1 | 15 | 293.5 | 50.1 | 194 |
| RAB22A | RAB22A | 20q13.32 | 15 | 220.1 | 22.8 | 22.8 |
| Radixin | RDX | 11q23 | 15 | 283 | 57.8 | 223.8 |
| RAN, member RAS oncogene family | RAN | 6p21 | 16 | 227.2 | 21.2 | 79.4 |
| Rhodanese;thiosulfate sulfurtransferase | TST | 22q13.1 | 14 | 199.7 | 19.2 | 66.5 |
| Ribosomal protein L13 | RPL13 | 16q24.3 | 13 | 379.9 | 68.9 | 248.4 |
| Ribosomal protein L27a | RPL27A | 11p15 | 15 | 272.3 | 44.8 | 173.2 |
| Ribosomal protein L4 | RPL4 | 15q22 | 16 | 230 | 24.2 | 97 |
| Ribosomal protein S18 | RPS18 | 6p21.3 | 16 | 290.7 | 27 | 107.8 |
| Ribosomal protein S29 | RPS29 | 14q21.3 | 15 | 306.4 | 56.6 | 219 |

**Table 3**

| **Colon cancer-related proteins (Up-regulated proteins)** | Gene Symbol | Gene Map | 150%≦ | Mean | SEM | SD |
|---|---|---|---|---|---|---|
| Ribosome binding protein 1 | RRBP1 | 20p12 | 15 | 202.4 | 16.4 | 63.6 |
| S adenosylhomocysteine hydrolase | AHCY | 20cen-q13.1 | 13 | 269.5 | 40.3 | 145.4 |
| Solute carrier family 25 (mitochondrial carrier; adenine nucleotide translocator), member 5 | SLC25A5 | Xq24-q26 | 13 | 229.8 | 36.7 | 132.4 |
| Solute carrier family 3(activator of dibasic and neutral amino acid transport), member 2 | SLC3A2 | 11q13 | 21 | 272.7 | 50.9 | 72.3 |
| Splicing factor 3B, subunit 3 | SF3B3 | 16q22.1 | 18 | 293.5 | 58 | 232.1 |
| Splicing factor, arginine/serine-rich 3 (sup20) | SFRS3 | 6p21 | 18 | 261.4 | 26.1 | 110.7 |
| U5 snRNP-specific protein, 116 kD | - | 17q21.31 | 15 | 239.6 | 25.2 | 97.4 |
| Ubiquitin isopeptidase T | USP13 | 3q26.2-q26.3 | 15 | 219.7 | 25.3 | 97.9 |
| Vitronectin | VTN | 17q11 | 14 | 218.2 | 37.7 | 141.2 |
| XTP3 transactivatied protein A | - | 16pal 1.2 | 15 | 281.8 | 38.1 | 147.6 |
| Galectin 1 | LGALS1 | 22q12-q13.1 | 19 | 218.3 | 16.6 | 72.3 |
| Reticulocalbin | RAN1 | 11p13 | 15 | 433 | 70.4 | 272.5 |
| Vimentin | VIM | 10p13 | 16 | 281.6 | 28.2 | 78.4 |
| ESP-2 (zyxin) | ZYX | 7q32-q35 | 13 | 226.7 | 23 | 82.9 |

**Table 4**

| **All or None (proteins) specifically expressed in cancer tissue)** | Gene Symbol | Gene Map | 150%≦ |
|---|---|---|---|
| Protein tyrosine phosphatase receptor type C | PTPRC | 1 q31-q32 | 17 |
| Protein tyrosine phosphatase, receptor type, alpha | PTPRA | 20p13 | 17 |
| orosomucoid 2 | ORM2 | 9q34.1-q34.3 | 18 |
| Tumor rejection antigen 1 | TRA1 | 12q24.2-q24.3 | 17 |
| glycyl-tRNA synthetase | GARS | 7p15 | 13 |
| TLS protein | FUS | 16p11.2 | 14 |
| Ribonuclease RNase A family 3 | RNASE3 | 14q24-q31 | 14 |
| heterogeneous nuclear ribonucleoprotein H2 | HNRPH2 | Xq22 | 13 |

Tables 5 to 7 show the list of identified proteins (Down-regulated proteins) whose expression level in cancerous part was lower than that in non-cancerous part. For example, Carbonic Anhydrase Form B (averaged expression level: 0.11 time that in non-cancerous part), whose expression level was decreased in 1.5 samples out of the 24 samples, or the like were identified. Table 8 shows the list of proteins detected specifically (All or None) in non-cancerous part. In Tables 5 to 8, "<66%" represents the number of samples in which the abundance of a protein of interest in cancerous part was less than 66% of that in non-cancerous part.

**Table 5**

| **Colon cancer-related proteins (Down-regulated proteins)** | GeneSymbol | Gene Map | <66% | Mean | SEM | SD |
|---|---|---|---|---|---|---|
| ADP-ribosylation factor-like 10C | ARL10C | 3p26.1 | 15 | 41.5 | 5.2 | 20.2 |
| aldehyde dehydrogenase2 | ALDH2 | 12q24.2 | 14 | 46.2 | 4.8 | 17.8 |
| Aipha-actinin 4 | ACTN2 | 1 q42-q43, | 16 | 44.2 | 4.4 | 17.6 |
| Annexin A2 isoform 2 | ANXA2 | 15q21-q22 | 16 | 42 | 4.7 | 18.9 |
| ATP synthase, H+ transporting, mitochondrial F0 complex, subunit d isoform a | ATP5H | 17q25 | 18 | 41.5 | 4.9 | 20.8 |
| ATP-binding member 12 cassette transporter family A member 12 | ABCA12 | 2q34 | 18 | 44.7 | 3.7 | 15.7 |
| Calnexin | CANX | 5q35 | 16 | 31.6 | 4.2 | 16.7 |
| Carbonic Anhydrase Form B | CA2 | 8q22 | 15 | 11.8 | 3.1 | 11.8 |
| Carbonyl reductase 1 | CBR1 | 21q22.12 | 12 | 52.6 | 3.1 | 10.9 |
| Gathepsin S | CTSS | 1q21 | 13 | 45.6 | 4.4 | 15.9 |
| cysteine rich protein 1 | CSRP1 | 1q32 | 14 | 31.3 | 4.5 | 16.7 |
| Dynein light chain 1 | DNCL1 | 12q24.23 | 19 | 47.3 | 4.1 | 17.8 |
| Endoplasmic-reticulum-lumenal protein 28 | C12orf8 | 12q24.13 | 15 | 46.6 | 4 | 15.6 |
| Enoyl coenzyme hydrase,short chain 1 | ECHDC1 | 6q22.33 | 16 | 36.6 | 5.2 | 20.9 |
| Eukaryotic translation elongation factor 2 | EEF2 | 19pter-q12 | 20 | 39 | 3.5 | 15.6 |
| Filamin B | FLNB | 3p14.3 | 19 | 38.3 | 3.9 | 17.2 |
| gelsolin isoform a | GSN | 9q34 | 17 | 47.8 | 4.2 | 17.3 |

**Table 6**

| **Colon cancer-related proteins) (Down-regulated proteins)** | GeneSymbol | Gene Map | <66% | Mean | SEM | SD |
|---|---|---|---|---|---|---|
| Glucosamine-fructose-6-phosphate aminotransferase | GFPT1 | 2p13 | 15 | 43.8 | 5.2 | 20.2 |
| GTP-binding protein Rab3B | RAB3B | 1p32-p31 | 17 | 56 | 3.3 | 13.7 |
| Haptoglobin | HP | 16q22.1 | 20 | 51.5 | 4.4 | 19.6 |
| Heterogeneous nuclear ribonucleoprotein A2 | HNRPA2B1 | 7p15 | 15 | 48 | 4.7 | 18.2 |
| Hydroxymethylglutaryl-CoA synthase, mitochondrial | HMGCS2 | 1p13-p12 | 18 | 38.7 | 6.4 | 24.7 |
| Isocitrate dehydrogenase 1 | IDH1 | 2q33.3 | 17 | 17.2 | 5 | 20.6 |
| Lymphocyte cytosolic protein 1 | LCP1 | 13q14.3 | 14 | 46.8 | 5.6 | 21.1 |
| Major vault protein | MVP | 16p13.1-p11.2 | 14 | 49.6 | 3.9 | 14.5 |
| MGC15429 protein | - | 3p21.2 | 14 | 43 | 4.6 | 17.1 |
| MHC class I antigen | HLA-A | 6p21.3 | 15 | 50.2 | 3.1 | 12 |
| Myosin, heavy polypeptide 14 | MYH14 | 19q13.33 | 13 | 51.5 | 4.4 | 19.6 |
| Myozenin 3 | MYOZ3 | 5q33.1 | 13 | 54.2 | 5.2 | 18.8 |
| NADH Ubiquinone oxidoreductase subunit B13 | NDUFA5 | 7q32 | 19 | 43.9 | 4.8 | 20.9 |
| Normal mucosa of esophagus specific 1 | - | 15q21.1 | 13 | 41 | 3.5 | 16.7 |
| Olfactomedin 4 | OLFM4 | 13q14.2 | 15 | 39.1 | 4.9 | 19.2 |
| phosphoenolpyruvate calboxykinase 2 | PCK2 | 14q11.2 | 14 | 49.9 | 4.9 | 18.3 |
| Phosphoglycerate mutase 1 | PGAM1 | 10q25.3 | 14 | 28.9 | 5.1 | 19.1 |

**Table 7**

| **Colon cancer-related proteins (Down-regulated proteins)** | GeneSymbol | Gene Map | <66% | Mean | SEM | SD |
|---|---|---|---|---|---|---|
| Proline arginine-rich end leucine-rich repeat protein precursor | PRELP | 1q32 | 16 | 42 | 4.7 | 18.9 |
| Protein kinase C and casein kinase substrate in neurons 2 | PACSIN2 | 22q13 | 19 | 46.9 | 5 | 21.1 |
| Protein P97 | VCP | 9p13-p12 | 20 | 42.6 | 4.2 | 16.9 |
| Pyridoxine 5'-phosphate oxidase | PNPO | 17q21.32 | 18 | 41 | 6.6 | 28 |
| Raf kinase inhibitor protein | PBP | 12q24.23 | 23 | 44.4 | 3.6 | 17.2 |
| Ras associated protein Rab5B | RAB5B | 12q13 | 14 | 49 | 4.2 | 15.8 |
| Retinoblastoma binding protein 4 | RBBP4 | 1 p34.3 | 13 | 53.8 | 4.4 | 15.8 |
| succinate dehydrogenase complex,subunit A" flavoprotein | SDHA | 5p15 | 13 | 39.7 | 4.9 | 17.8 |
| Thioredoxin domain containing 5 | TXNDC5 | 6p24.3 | 17 | 40.9 | 4.7 | 19.3 |
| TNRC15 protein | TNRC15 | 2q37.1 | 17 | 35.9 | 3.7 | 15.3 |

**Table 8**

| **All or None (proteins spedifically expressed in normal tissue)** | Gene Symbol | Gene Map | <66% |
|---|---|---|---|
| Collagen, type XIV, alpha 1 | COL14A1 | 8q23 | 13 |
| Desmoglein 2 | DSG2 | 18q12.1 | 13 |

In the following Examples 2 and 3, 6 proteins out of the proteins whose expression level determined by the NBS method was higher in cancerous part than in non-cancerous part, i.e., ZYX (Zyxin), RAN (RAN, member RAS oncogene family), RCN1 (Reticulocalbin), AHCY (S-adenosylhomocysteine hydrolase), SGALS1 (Galectin1), and VIM (Vimentin), were analyzed by western blotting of tissues and validation analysis using immunohistochemical staining of tissues.

### <Example 2: Western Blotting Analysis>

Normal tissues (i.e., non-cancerous part of large-intestinal mucosal epithelial tissues) and cancerous tissues (i.e., cancerous part of large-intestinal mucosal epithelial tissues) sampled from colon cancer patients (5 patients) were provided. Each of the tissues was homogenized in a solubilizing buffer A (50 mM Tris-HCl (pH 8.0), 100 mM NaCl, 10 mM EDTA, protease inhibitor (aprotinin, PMSF, Leupeptin) solution), and was then ultracentrifuged at 100000 x G (4°C, 1 hour) to obtain a supernatant solution as a soluble fraction. Then, the precipitate obtained by centrifugation was again homogenized in a solubilizing buffer B (9M urea, 2% CHAPS, 10 mM EDTA, protease inhibitor (aprotinin, PMSF, Leupeptin) solution), and was then ultracentrifuged at 100000 x G (4°C, 1 hour) to obtain a supernatant solution as an insoluble fraction.

Among the thus obtained protein extract solutions, the soluble fractions were numbered patients 1 to 5 and used for analysis of ZYX, RAN, RCN1, and AHCY, and the insoluble fractions were numbered patients 6 to 10 and used for analysis of SGALS1 and VIM.

Each of the extract solutions was separated on a 12.5% acrylamide (15% acrylamide for only SGALS1 analysis) SDS-PAGE gel, and then transferred to a nitrocellulose membrane. Then, primary antibody (which will be described later) against each of the proteins of interest was loaded onto the transferred sample and incubated at room temperature for 2 hours. After the reaction, the nitrocellulose membrane was washed with a PBS (Phosphate Buffered Saline) buffer, and then secondary antibody (which will be described later) solutions were added to carry out reaction for 1 hour. After the reaction with antibodies, an ECL coloring solution was added to detect protein bands.

It is to be noted the following antibodies were used in the present Example.

### Primary Antibodies:

Mouse anti-human ZYX polyclonal antibody
(antibody dilution ratio 1/500)

Mouse anti-human RAN monoclonal antibody
(antibody dilution ratio 1/2000)

Mouse anti-human AHCY polyclonal antibody
(antibody dilution ratio 1/1000)

Rabbit anti-human RCN1 monoclonal antibody
(antibody dilution ratio 1/1000)

Rabbit anti-human SGAL1 polyclonal antibody
(antibody dilution ratio 1/1000)

Rabbit anti-human VIM polyclonal antibody
(antibody dilution ratio 1/1000)

### Secondary Antibodies:

Horseradish peroxidase conjugated-sheep antimouse IgG antibody (antibody dilution ratio 1/400)
Horseradish peroxidase conjugated-donkey anti-rabbit IgG antibody (antibody dilution ratio 1/400)

Fig. 1 shows electrophoretic patterns obtained by the above-described western blotting analysis. In Fig. 1, "N" represents a non-cancerous part-derived sample and "T" represents a cancerous part-derived sample.

As can be seen from Fig. 1, expression of all the 6 proteins was increased in cancerous part in all the 5 patients. That is, the proteins which showed higher expression in cancerous part than in non-cancerous part in the quantitative analysis by the NBS method in Example 1 actually showed higher expression in cancerous part than in non-cancerous part also in the quantitative analysis by western blotting. This indicates that there is a good correlation between the result of the quantitative analysis by the NBS method and the result of the quantitative analysis by western blotting. From the result, it has been confirmed that these molecules are proteins associated with colon cancer.

From the findings, it can be easily estimated that the proteins which showed higher expression in non-cancerous part than in cancerous part in the quantitative analysis by the NBS method, (i.e., the proteins whose expression in cancerous part was suppressed as compared to that in non-cancerous part) will show the same expression tendency also in the quantitative analysis by western blotting.

### <Example 3: Analysis by Immunohistochemical Staining>

Paraffin-embedded sections (4 µm) of normal tissues (i.e., non-cancerous part of large-intestinal mucosal epithelial tissues) and cancerous tissues (i.e., cancerous part of large-intestinal mucosal epithelial tissues) which were sampled from colon cancer patients (10 patients) were provided. As for antibodies, the same ones as used in the above-described western blotting analysis were used.

The paraffin section was reacted with a primary antibody at room temperature for 1 hour, and was then washed with a PBS buffer and further reacted with a streptavidin-biotin peroxidase complex. Finally, the tissue section was reacted with a coloring solution (3,3'-diaminobenzadinetetrahydrochloride, 0.01% peroxidase, 0.05M tris aqueous solution (pH 7.6)) for 3 minutes to carry out histological staining. The results are shown in Fig. 2.

In Fig. 2, (A), (B), (C), (D), (E) and (F) show the results of immunohistochemical staining for ZYX, RAN, RCN1, AHCY, SGALS1, and VIM, respectively, and "T" represents the result of staining of cancerous part and "N" represents the result of staining of non-cancerous part.

As shown in Fig. 2, it was confirmed that the expression of all the 6 proteins was increased in cancerous cells in cancerous part (or in interstitial cells surrounding the cancerous cells) in all the colon cancer patients. That is, the proteins which showed higher expression in cancerous part than in non-cancerous part in the quantitative analysis by the NBS method in Example 1 actually showed higher expression in cancerous part than in non-cancerous part also in the analysis by immunohistochemical staining. This indicates that there is a good correlation between the result of the quantitative analysis by the NBS method and the result of the analysis by immunohistochemical staining. From the result, it has been confirmed that these molecules are proteins associated with colon cancer.

From the findings, it can be easily estimated that the proteins which showed higher expression in non-cancerous tissues part than in cancerous part in the quantitative analysis by the NBS method (i.e., the proteins whose expression in cancerous part was suppressed as compared to that in non-cancerous part) will show the same expression tendency also in the analysis by immunohistochemical staining.

As described above, the proteins as claimed in claim 1 express at a higher level in colon cancer tissues than in non-cancerous tissues, and on the other hand, the proteins as claimed in claim 3 express at a lower level in colon cancer tissues than in non-cancerous tissues. It is clear that these proteins can be used as tumor markers for colon cancer. In a case where an examination is performed to identify the morbidity of colon cancer for a sample which is unknown about the morbidity of colon cancer using such a protein as a tumor marker for colon cancer, the methods described in Examples 1 to 3 may be used.

The Examples described above show concrete embodiments within the scope of the present invention, but the present invention is not limited to these Examples and may be implemented in various embodiments. Therefore, the Examples described above are merely illustrative in every respect, and should not be construed as being restrictive. Further, the changes that fall within the equivalents of the claims are all within the scope of the present invention.

## Claims

1. A tumor marker for colon cancer including at least one protein selected from the group consisting of:
6-phosphogluconolactonase; Alpha1 acid glyco protein; Alpha-actinin 1; Apurinic endonuclease; Calumenin; Chaperonin; Clathrin heavy chain 1; Clathrin light polypeptide A; c-myc binding protein; Complement factor H; Cysteine rich intestinal protein 1; F-box protein 40; Fibrinogen gamma; Fk506 Binding Protein Fkbp Mutant R42kH87V COMPLEX WITH Immunosuppressant Fk506; Guanine nucleotide binding protein (G protein), beta polypeptide 2-like 1; Heat shock 70kD protein 9B; Heparan sulfate proteoglycan 2; HLA-C; hypothetical protein FLJ38663; L-plastin polypeptide; MBC2; Migration inhibitory factor-related protein 14 variant E; Mitogen inducible gene; Proteasome subunit p58; RAB18, member RAS oncogene family; RAB22A; Radixin; RAN, member RAS oncogene family; Rhodanese;thiosulfate sulfurtransferase; Ribosomal protein L13; Ribosomal protein L27a; Ribosomal protein L4; Ribosomal protein S18; Ribosomal protein S29; Ribosome binding protein 1; S adenosylhomocysteine hydrolase; Solute carrier family 25 (mitochondrial carrier; adenine nucleotide translocator), member 5; Solute carrier family 3(activator of dibasic and neutral amino acid transport), member 2; Splicing factor 3B, subunit 3; Splicing factor, arginine/serine-rich 3 (SRp20); U5 snRNP-specific protein, 116 kD; Ubiquitin isopeptidase T; Vitronectin; XTP3 transactivatied protein A; Galectin 1; Reticulocalbin 1; Vimentin; ESP-2 (zyxin); Protein tyrosine phosphatase receptor type C; Protein tyrosine phosphatase, receptor type, alpha; orosomucoid 2; Tumor rejection antigen 1; glycyl-tRNA synthetase; TLS protein; Ribonuclease RNase A family 3; and heterogeneous nuclear ribonucleoprotein H2.

2. A tumor marker for colon cancer including a protein selected from the group consisting of:
ADP-ribosylation factor-like 10C; aldehyde dehydrogenase2; Alpha-actinin 4; Annexin A2 isoform 2; ATP synthase, H+ transporting, mitochondrial F0 complex, subunit d isoform a; ATP-binding cassette transporter family A member 12; Calnexin; Carbonic Anhydrase Form B; Carbonyl reductase 1; Cathepsin S; cysteine rich protein 1; Dynein light chain 1; Endoplasmic-reticulum-lumenal protein 28; Enoyl coenzyme hydrase,short chain1; Eukaryotic translation elongation factor 2; Filamin B; gelsolin isoform a; Glucosamine-fructose-6-phosphate aminotransferase; GTP-binding protein Rab3B; Haptoglobin; Heterogeneous nuclear ribonucleoprotein A2; Hydroxymethylglutaryl-CoA synthase, mitochondrial; Isocitrate dehydrogenase 1; Lymphocyte cytosolic protein 1; Major vault protein; MGC15429 protein; MHC class I antigen; Myosin, heavy polypeptide 14; Myozenin 3; NADH Ubiquinone oxidoreductase subunit B13; Normal mucosa of esophagus specific 1; Olfactomedin 4; phosphoenolpyruvate calboxykinase 2; Phosphoglycerate mutase 1; Proline arginine-rich end leucine-rich repeat protein precursor; Protein kinase C and casein kinase substrate in neurons 2; Protein P97; Pyridoxine 5'-phosphate oxidase; Raf kinase inhibitor protein; Ras associated protein Rab5B; Retinoblastoma binding protein 4; succinate dehydrogenase complex,subunit A,flavoprotein; Thioredoxin domain containing 5; TNRC15 protein;
Collagen, type XIV, alpha 1, and Desmoglein 2.

3. A method for identifying the morbidity of colon cancer by using, as a tumor marker for colon cancer, at least one protein selected from the group consisting of:
6-phosphogluconolactonase; Alpha1 acid glyco protein; Alpha-actinin 1; Apurinic endonuclease; Calumenin; Chaperonin; Clathrin heavy chain 1; Clathrin light polypeptide A; c-myc binding protein; Complement factor H; Cysteine rich intestinal protein 1; F-box protein 40; Fibrinogen gamma; Fk506 Binding Protein Fkbp Mutant R42kH87V COMPLEX WITH Immunosuppressant Fk506; Guanine nucleotide binding protein (G protein), beta polypeptide 2-like 1; Heat shock 70kD protein 9B; Heparan sulfate proteoglycan 2; HLA-C; hypothetical protein FLJ38663; L-plastin polypeptide; MBC2; Migration inhibitory factor-related protein 14 variant E; Mitogen inducible gene; Proteasome subunit p58; RAB18, member RAS oncogene family; RAB22A; Radixin; RAN, member RAS oncogene family; Rhodanese;thiosulfate sulfurtransferase; Ribosomal protein L13; Ribosomal protein L27a; Ribosomal protein L4; Ribosomal protein S18; Ribosomal protein S29; Ribosome binding protein 1; S adenosylhomocysteine hydrolase; Solute carrier family 25 (mitochondrial carrier; adenine nucleotide translocator), member 5; Solute carrier family 3(activator of dibasic and neutral amino acid transport), member 2; Splicing factor 3B, subunit 3; Splicing factor, arginine/serine-rich 3 (SRp20); U5 snRNP-specific protein, 116 kD; Ubiquitin isopeptidase T; Vitronectin; XTP3 transactivatied protein A; Galectin 1; Reticulocalbin 1; Vimentin; ESP-2 (zyxin); Protein tyrosine phosphatase receptor type C; Protein tyrosine phosphatase, receptor type, alpha; orosomucoid 2; Tumor rejection antigen 1; glycyl-tRNA synthetase; TLS protein; Ribonuclease RNase A family 3; and heterogeneous nuclear ribonucleoprotein H2.

4. The method for identifying the morbidity of colon cancer according to claim 3, including:
measuring the level of the protein in a sample derived from a person of interest who should be examined to identify the morbidity of colon cancer; and
comparing the measured level to the normal level of the protein,
wherein a higher measured level than the normal level is used as one indicator indicating that there is a high possibility that the person of interest has colon cancer.

5. A method for identifying the morbidity of colon cancer by using, as a tumor marker for colon cancer, a protein selected from the group consisting of ADP-ribosylation factor-like 10C; aldehyde dehydrogenase2; Alpha-actinin 4; Annexin A2 isoform 2; ATP synthase, H+ transporting, mitochondrial F0 complex, subunit d isoform a; ATP-binding cassette transporter family A member 12; Calnexin; Carbonic Anhydrase Form B; Carbonyl reductase 1; Cathepsin S; cysteine rich protein 1; Dynein light chain 1; Endoplasmic-reticulum-lumenal protein 28; Enoyl coenzyme hydrase,short chain1; Eukaryotic translation elongation factor 2; Filamin B; gelsolin isoform a; Glucosamine-fructose-6-phosphate aminotransferase; GTP-binding protein Rab3B; Haptoglobin; Heterogeneous nuclear ribonucleoprotein A2; Hydroxymethylglutaryl-CoA synthase, mitochondrial; Isocitrate dehydrogenase 1; Lymphocyte cytosolic protein 1; Major vault protein; MGC15429 protein; MHC class I antigen; Myosin, heavy polypeptide 14; Myozenin 3; NADH Ubiquinone oxidoreductase subunit B13; Normal mucosa of esophagus specific 1; Olfactomedin 4; phosphoenolpyruvate calboxykinase 2; Phosphoglycerate mutase 1; Proline arginine-rich end leucine-rich repeat protein precursor; Protein kinase C and casein kinase substrate in neurons 2; Protein P97; Pyridoxine 5'-phosphate oxidase; Raf kinase inhibitor protein; Ras associated protein Rab5B; Retinoblastoma binding protein 4; succinate dehydrogenase complex,subunit A, flavoprotein; Thioredoxin domain containing 5; TNRC15 protein;
Collagen, type XIV, alpha 1, and Desmoglein 2.

6. The method for identifying the morbidity of colon cancer according to claim 5, including:
measuring the level of the protein in a sample derived from a person of interest who should be examined to identify the morbidity of colon cancer; and
comparing the measured level to the normal level of the protein,
wherein a lower measured level than the normal level is used as one indicator indicating that there is a high possibility that the person of interest has colon cancer.
